# EUROPÄISCHE PATENTSCHRIFT

(11) **EP 3 188 803 B1**
(45) Veröffentlichungstag und Bekanntmachung des Hinweises auf die Patenterteilung: **06.10.2021**
(21) Anmeldenummer: 15750758.3
(22) Anmeldetag: 18.08.2015
(51) Int. Cl.: A61Q 19/10, A61K 8/03, A61K 8/04

(54) **TRANSPARENTES MEHRPHASENSYSTEM**
TRANSPARENT MULTI-PHASE SYSTEM
SYSTÈME MULTIPHASÉ TRANSPARENT

(30) Priorität: 02.09.2014 DE 102014217512; 03.09.2014 DE 102014217530
(43) Veröffentlichungstag der Anmeldung: 12.07.2017
(73) Patentinhaber: Beiersdorf AG, 20253 Hamburg (DE)
(72) Erfinder: TREU, Jens, 22844 Norderstedt (DE); FISCHER, Britta, 22880 Wedel (DE); KÖNIG, Sylvia, 21635 Jork (DE); ARGEMBEAUX, Horst, 21465 Wentorf (DE)
(74) Vertreter: Beiersdorf AG
(86) Internationale Anmeldenummer: PCT/EP2015/068904
(87) Internationale Veröffentlichungsnummer: WO 2016/034398

(56) Entgegenhaltungen:
- EP-A1- 1 316 300
- EP-A2- 1 108 421
- EP-A2- 1 169 998
- WO-A1-2007/052230

## Beschreibung

Die Erfindung ist eine Kombination aus einer niedrigviskosen kosmetischen oder dermatologischen Mehrphasenreinigungszubereitung in einem transparenten Aerosolbehältnis.

Um ein Zwei-Phasenprodukt zu bilden, bedarf es einer Phase mit Wasser und einer Phase mit Öl. Im Ruhezustand sind diese beiden Phasen idealerweise optisch klar voneinander getrennt zu erkennen. Durch die unterschiedlichen Dichten vermischen sich diese beiden Phasen nicht selbstständig miteinander und bilden dadurch ein horizontal getrenntes 2-phasiges System aus. Durch das Einbringen von Energie z.B. durch Schütteln, kann man die Phasen kurzzeitig miteinander vermischen. Aufgrund des Dichteunterschiedes und einer hohen Grenzflächenspannung (GFS) von Öl gegenüber Wasser kommt es allerdings danach wieder zu einer sehr schnellen Auftrennung in die zwei Phasen.

Die 2-Phasigkeit wird dadurch erreicht, dass man ölige und wässrige Bestandteile in einer Zubereitung umfasst, die durch gezielte Auswahl von oberflächenaktiven Substanzen, wie Emulgatoren oder Tensiden, nicht ausreichend stabilisiert werden. Durch die Zugabe von Emulgatoren/Tensiden könnte ansonsten eine Emulsion erzeugt werden und eine Zwei- oder Mehrphasigkeit damit unmöglich werden.

Bekannte einphasige Emulsionszubereitungen, wie in der DE 19537836 A1 beschrieben, sind daher keine Mehrphasenzubereitungen entsprechen der vorliegenden Erfindung.

Im Bereich der Reinigungsprodukte hat sich gezeigt, dass zweiphasige Systeme eine bessere Reinigungsleistung haben, besonders bei wasserfestem Make-up. Hier wird die gut reinigende Wirkung von Ölen mit Wasser kombiniert, welches die ölige Sensorik minimiert.

Kosmetische Zweiphasenprodukte sind beispielsweise im Bereich der Haarpflege und -styling oder als Make-up Entferner bekannt.

Bei der Anwendung als Reinigungsprodukt, beispielsweise als Duschprodukt, zeigen sich bei den Mehrphasigen-Produkten bislang jedoch einige Nachteile.

So sind Mehr- und insbesondere 2-phasige Systeme sehr niedrigviskos formuliert, so dass derartige Produkte für eine Anwendung unter der Dusche ungeeignet sind, da das Produkt vor der Applikation aufgrund der niedrigen Viskosität durch die Finger rinnt.

Als niedrigviskos im Bezug auf Duschprodukte werden dynamische Viskositäten im Bereich unterhalb von 1500 mPa^{*}s angesehen, vorzugsweise weniger als 1000 mPas besonders bevorzugt im Bereich 500 bis 800 mPas.

Dem gegenüber hat ein Standard-Duschprodukt, welches weitgehend ölfrei ist, eine Viskosität von ca. 3000 bis 4000 mPa^{*}s. Dadurch ist es dem Verbraucher möglich, diese Duschprodukte unter der Dusche bequem anzuwenden. Es rinnt nicht durch die Finger und lässt sich gut aufschäumen. Allerdings verhindert die höhere Viskosität die Bildung eines 2-Phasen Systems, da eine Durchmischung der Phasen durch schütteln und eine saubere Trennung aufgrund der hohen Viskosität nicht möglich ist.

Ein weiteres Problem ergibt sich aufgrund des Ölgehaltes mehrphasiger Produkte. So zeichnen sich 2- phasige Produkte aufgrund einer wässrigen Tensid- und einer pflegenden Ölphase durch eine besondere Pflegeleistung aus.

Dem Fachmann ist aber bekannt, dass Öle das Schaumverhalten einer Zubereitung negativ beeinflussen, da sie eher entschäumend wirken. Die entschäumende Wirkung der Öle beruht darauf, dass sie aufgrund ihrer Unlöslichkeit und ihrer Oberflächenaktivität an die Grenzfläche Flüssigkeit/Luft wandern und in diese eindringen. Zudem werden an der Phasengrenzfläche vorhandene Tenside von den Ölen gebunden und stehen damit zur Emulsionsbildung nicht mehr zur Verfügung.

Eine ölhaltige 2-Phasenzubereitung, die aufgeschäumt appliziert werden kann, ist daher nur schwer formulierbar.

In der WO 2002015849 wird dementsprechend nur eine ölfreie 2-Phasenreinigungs-zubereitung offenbart. Zudem umfasst die Zubereitung zwingend Verdicker und Mineralsalze.

EP 1108421 A2 beschreibt schäumbare Mehrphasen-Reinigungszubereitungen, wobei zwingend auf Treibgase verzichtet wird.

Ebenso werden in der DE 10159002 A1 Mehrphasen-Sonnenschutzzubereitungen beschrieben, bei denen auf Treibgase verzichten wird.

In der DE 3628531 A1 werden verschäumbare Cremes aus einer Creme-Emulsion und einem Treibmittel beschrieben. Es wird hierbei explizit auf den Einfluss der Einzelbestandteile sowie der Viskosität der Emulsion auf die Stabilität des erzeugten Schaumes hingewiesen. Die Zubereitungen der DE 3628531 A1 sind daher keine Mehrphasenzubereitungen und umfassen zwingend nicht-ionische Tenside.

Viskositäten von 110 mPa^{*}s werden hierin als zu flüssig bezeichnet um einen stabilen Schaum erzeugen zu können.

DE 10029262 A1 offenbart Aerosol-Körperlotionen umfassend Verdickungsmittel. Eine niedrige Viskosität von 200 - 2000 mPa^{*}s wird erst durch nach dem Mischen mit Treibgasen erreicht. Ein Alkohol - (Ethanol) - gehalt von 20 bis 80% ist ebenso bevorzugt.

Weitere Probleme schäumbarer Zubereitungen ergeben sich dadurch, dass Duschprodukte in Aerosol-Verpackungen oft eine Inkompatibilität zeigen, z.B. Schädigung des Innenschutz-Lackes und dadurch Reaktion zwischen Füllgut und Alu- oder Weißblechdose, die zu Korrosion führen kann.

Damit ist ein Sicherheitsrisiko für den Verbraucher gegeben, da es zu Undichtigkeiten oder auch Bersten der Druckgasverpackung kommen kann und auch durch die Reaktion mit Metall eine Verunreinigung des Endproduktes erfolgt.

Zusätzlich ist bei den herkömmlichen Druckgasbehältnissen aus Aluminium, Weißblech oder anderen Metallen die optisch attraktive 2-Phasigkeit, die auch ein Ausdruck der Pflegeleistung des Produktes für den Konsumenten darstellt, nicht sichtbar.

Weiterhin werden Reinigungsprodukte in Kontakt mit Wasser angewendet, z.B. unter der Dusche, so dass auch hier eine Metallverpackung ungeeignet ist.

Daher wäre es wünschenswert eine 2-phasige Zubereitung und Applikationsform zu finden, die sowohl die Probleme der niedrigen Viskosität, der Schäumbarkeit als auch die der metallenen Verpackung löst und die Transparenz des Packmittels ermöglicht.

Mit dem Stand der Technik, hochviskose Produkte und nichttransparenten Alu- oder Weißblechdose-Aerosoldosen, sind diese Probleme nicht zu lösen.

Die Erfindung ist eine kosmetische oder dermatologische Mehrphasenzubereitung. Die Mehrphasenzubereitung umfasst mindestens eine Ölphase und mindestens ein Wasserphase, die wiederum ein oder mehrere Tenside umfasst. Die Viskosität jeder Einzelphase der Zubereitung weist insgesamt eine Viskosität im Bereich von 20 bis 300 mPa^{*}s auf, vorteilhaft im Bereich von 30 bis 150 mPa^{*}s, insbesondere weniger als 100 mPa^{*}s. Damit liegen alle Viskositäten der Gesamtzubereitung sowie der Einzelphasen, vor dem Vermischen und Ausbringen, im erfindungsgemäßen Bereichen von 20 bis 300 mPa^{*}s.

Die Erfindung ist darüber hinaus ein Kit, eine Kombination umfassend ein Behältnis und diese kosmetische oder dermatologische Mehrphasenzubereitung.

Als Viskosität wird die dynamische Viskosität der ungeschäumten Mehrphasenzubereitung, bzw. der unvermischten Einzelphasen betrachtet. Die Viskosität in mPa* s wird beispielsweise mittels Rheomat R123, Messkörper 3 bei 25°C ermittelt (Viskosimeter: Rheomat 123; Messkörper 3 (ein rotational Rheometer zur Messung der Viskosität mit VT02-measuring systems).

Die Zubereitung ist bevorzugt eine Zweiphasigen-Zubereitung. D.h. die Mehrphasenzubereitung besteht aus zwei Phasen. Bei Hinzunahme der Treibgase bildet die Treibgasphase ggf. eine dann dritte Phase, die aber nicht unter dem Begriff der Mehrphase zu zählen ist. Weiter bevorzugt ist die Anwendung der erfindungsgemäßen Zubereitung als Reinigungsprodukt, bevorzugt als Duschprodukt oder als Gesichtsreinigung. Eine weitere mögliche und bevorzugte Anwendungsform der erfindungsgemäßen Zubereitung ist ein Gesichtsreinigungsschaum, der beispielsweise auch außerhalb einer Dusche angewandt werden kann.

Die erfindungsgemäße Zubereitung liegt vorzugsweise in einem transparenten Behältnis vor in dem ein oder mehrere Treibgase enthalten sind, die mit der Zubereitung in Kontakt stehen. Transparenter Behälter bedeutet, dass dessen Wandung eine Transmission für sichtbares Licht von 25% bis 100 % aufweist.

Die Zubereitung kann über eine Ausbringvorrichtung aus dem Behältnis ausgebracht werden.

Die niedrige Viskosität ermöglicht, dass sich die Phasen beispielsweise beim Schütteln ausreichend homogen mischen lassen und dann wieder in einer angemessenen Zeit trennen können. So kann ein mehr-, bevorzugt zweiphasiges Produkt bereitgestellt werden.

Die vorliegende Mehrphasenzubereitung ist dabei keine Emulsion, sondern durch optisch klar durch getrennte Phasen charakterisiert.

Formeln mit einer solch geringen Viskosität lassen sich jedoch wie erläutert nicht unter der Dusche applizieren, daher muss das Produkt vor der Verwendung stark aufgeschäumt werden. Dies kann manuell nicht erzeugt werden, da das Produkt zu schnell durch die Finger rinnt.

Durch die Abfüllung eines erfindungsgemäßen Produktes in ein Aerosol wird dadurch das Produkt bei der Applikation stark aufgeschäumt und kann so problemlos appliziert werden, ohne dass es durch die Finger rinnt.

Durch die Transparenz des Behältnisses und 2-Phasigkeit des erfindungsgemäßen Produktes wird die Pflege im Gesamtprodukt für den Konsumenten sichtbar. Außerdem kann durch das transparente Packmittel erst erkannt werden, wann das Produkt vor der Applikation genügend geschüttelt und bereit zur Anwendung ist. Dies ist kein rein ästhetischer Vorteil sondern ein insbesondere für die Anwendung technisch wichtiges Merkmal.

Das erfindungsgemäße Kit umfasst somit sowohl die Vorteile der niedrigen Viskosität der Mehrphasenzubereitungen mit den Vorzügen der treibgashaltigen Verpackung.

Die Kombination ermöglicht in vorteilhafterweise die Anwendung eines Reinigungsproduktes z.B. unter Dusche.

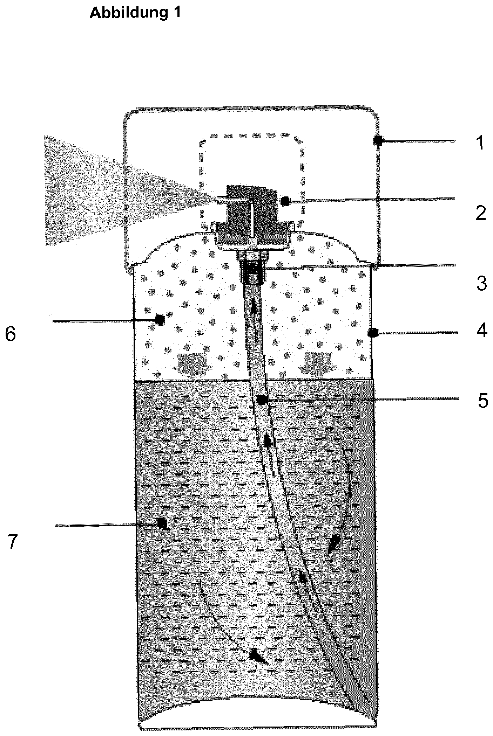

In Abbildung 1 ist der Aufbau eines erfindungsgemäßen Produktes schematisch dargestellt. Die Zubereitung, das Füllgut (7), auch Wirkstofflösung genannt, wird in die Aerosoldose (4) eingefüllt, mit dem Ventilteller (3) verschlossen (geclincht/gecrimpt) und dann mit einem flüssigen Treibgasgemisch (6) begast. Das Treibgas wird unter hohem Druck verflüssigt, z.B. Propan/Butan/Isobutan in den Druckstufen 2,7 bar, 3,2 bar oder 3,5 bar.

Das Treibmittel (6) ist ständig bestrebt wieder in seinen gasförmigen Zustand zurückzukehren und hält somit den Druck in der Dose aufrecht. Durch Schütteln werden Treibgas (6) und das zweiphasige Füllgut (7) vermischt. Die mehrphasige Zubereitung (7) ist in Abbildung 1 dabei im bereits vermischten Zustand dargestellt.

Durch das Auslösen des entsprechenden Sprühkopfes (2) entweicht das Gemisch durch ein Steigrohr (5) über das Ventil (3). Das Gas kann sich nun wieder ausdehnen, es bilden sich kleine Luftblasen in der Wirkstofflösung, wodurch ein Schaum erzeugt wird. Schäume benötigen dabei keinen hohen Treibgasanteil, da höhere Treibgasanteile für eine Aufspaltung des Füllguts in kleinere und feinere Tröpfchen führen würden und der Inhalt dann ganz fein vernebelt ausgetragen würde.

Durch das Treibgas wird das Öl/Wasser/Tensidgemisch so stark aufgeschäumt, dass dieser negative Einfluss überwunden wird.

Weiterhin vergrößert das Treibgas die Grenzfläche und verstärkt die emulgierende Wirkung der Tenside, wodurch durch diese Aerosoltechnologie ein ausreichend stabiler und sehr cremiger Schaum ermöglicht wird.

Zusätzlich kann erfindungsgemäß ein deutlich höherer Ölanteil eingebracht werden im Vergleich zum Standard-Duschprodukt und dadurch wird ein hautpflegenderes Duschprodukt zur Verfügung gestellt.

Das Behältnis enthält oder besteht bevorzugt aus Polyester, insbesondere Polyethylenterephthalat (PET). Polyethylenterephthalat (Kurzzeichen PET) ist ein durch Polykondensation hergestellter thermoplastischer Kunststoff aus der Familie der Polyester.

Bei transparenten PET-Aerosole zeigen sich oft Probleme. Beispielsweise durch starke Hitzeeinwirkung kann der Behälter schmelzen oder alkoholhaltige Füllgüter können sich entzünden.

Erfindungsgemäß bevorzugt sind die erfindungsgemäßen Zubereitungen daher alkoholfrei.

Die erfindungsgemäßen Zubereitungen sind vorteilhaft frei von einwertigen Alkoholen. D.h. der Anteil an Alkohol beträgt weniger als 1 Gew.%, insbesondere weniger als 0,1 Gew.%, bevorzugt 0 Gew.%, bezogen auf die Gesamtmasse der Zubereitung. Erfindungsgemäß sind unter dem Begriff Alkohol nicht die als Polyole bekannten Verbindungen zu verstehen, wie beispielsweise Glycerin. Alkohole auf die verzichten werden können, sind erfindungsgemäß die einwertigen Alkohole, Verbindungen mit nur einer Hydroxydgruppe pro Molekül, wie insbesondere Ethanol, Propanol und Isopropanol.

Ebenso wird der Anteil an Treibgasen erfindungsgemäß vorteilhaft im Bereich von 3 bis 10 Gew.%, insbesondere im Bereich von 4 bis 8 Gew.%, bezogen auf die Gesamtmasse der Zubereitung inklusive der Treibgase, gewählt. In diesem Bereich ist die Gefahr der Brennbarkeit vermindert. Zudem handelt es sich erfindungsgemäß um wässrige Zubereitungen und der Wasseranteil wird bevorzugt im Bereich über 50 Gew.%, bezogen auf die Gesamtmasse der Zubereitung ohne Treibgase, was wiederum zu einer verminderten Gefahr der Brennbarkeit führt.

Als Treibgase werden Propan oder Butan oder deren Gemische gewählt, wie beispielsweise Propan, Butan und/oder Isobutan verwendet.

Der Anteil der Ölphase wird vorteilhaft im Bereich bis zu 20 Gew.%, bezogen auf die Gesamtmasse der Zubereitung ohne Treibgase, gewählt.

Als bevorzugte Öle werden ein oder mehrere Öle aus der Gruppe Paraffinum Liquidum, Isohexadecane, Isopropyl Palmitate, C12-15 Alkyl Benzoate und/oder C12-13 Alkyl Lactate gewählt.

Der Anteil an Tensiden wird vorteilhaft im Bereich bis zu 20 Gew.%, bezogen auf die Gesamtmasse der Zubereitung ohne Treibgas, gewählt.

Als Tenside werden Tenside aus der Gruppe der anionischen und amphoteren Tenside gewählt, insbesondere Tenside aus der Gruppe Cocamidopropyl Betaine, Sodium Laureth Sulfate, Sodium Cocoamphoacetate, Sodium Myreth Sulfate, Coco-Glucoside, Lauryl Glucoside, Disodium PEG-5 Laurylcitrate Sulfosuccinate und/oder Disodium Laureth Sulfosuccinate.

Es wird eine Kombination aus anionischen und amphoteren Tensid eingesetzt, vorzugsweise Sodium Laureth Sulfate und Cocamidopropyl Betaine.

Bevorzugt werden als Tenside nur Tenside aus der Gruppe der anionischen und amphoteren Tenside gewählt, insbesondere nur die zuvor als bevorzugt identifizierten Tenside.

Vorteilhaft wird der Zubereitung Propylenglykol zugesetzt.

Propylenglykol kommt als sogenanntes Kotensid in Mehrkomponentensystemen zur Anwendung. Es kann Emulsionsbildung begünstigen und die Löslichkeit verschiedener Stoffe deutlich verbessern und eine stabilere Dispersion gewährleisten. Durch die Zugabe des Propylenglykols in die erfindungsgemäße Zubereitung wird eine ausreichend lange Durchmischung der Öl-/wässrigen Tensidphase erreicht.

Die Zubereitung umfasst bevorzugt Propylenglykol, bevorzugt zu einem Anteil von bis 10 Gew.%, insbesondere im Bereich um 5 Gew.%, bezogen auf die Gesamtmasse der Zubereitung.

Die Auswahl an Tensiden und/oder Propylenglykol zeigt die Möglichkeit der Bereitstellung einer Mehrphasen-, insbesondere Zweiphasen Reinigungszubereitung mit geringer Viskosität.

Bevorzugt sind die Mischungsverhältnisse der Tenside, Öle und Wasser im Bereich von 0,8 bis 2,5 zu 0,8 bis 2,5 zu 2,5 bis 8 zu wählen. Insbesondere sind die Verhältnisse im Bereich von 1 bis 2 (Tenside) zu 1 bis 2 (Öle) zu 3 bis 7 (Wasser) zu wählen oder wie folgende Tabelle angibt:

| **A** | **B** | **C** | **D** |
|---|---|---|---|
| 16 Teile Öl | 15 Teile Öl | 10 Teile Öl | 20 Teile Öl |
| 17 Teile Tensid | 15 Teile Tensid | 20 Teile Tensid | 20 Teile Tensid |
| 67 Teile Wasser | 70 Teile Wasser | 70 Teile Wasser | 60 Teile Wasser |

Die Verhältnisse beziehen sich auf die Gewichtsanteile ohne Treibgase.

Bevorzugt sind in der Wasserphase 3 bis 8 Teile, insbesondere 5 Teile Propylenglykol vorhanden.

Die erfindungsgemäße Kombination, Kit, umfasst in einer bevorzugten Ausführungsform demnach eine kosmetische 2-Phasenzubereitung, deren Ölphase ein oder mehrere Öle aus der Gruppe Paraffinum Liquidum, Isohexadecane, Isopropyl Palmitate, C12-15 Alkyl Benzoat, Sonneblumenöl und/oder C12-13 Alkyl Lactate enthält. Die Wasserphase umfasst ein oder mehrere Tenside aus der Gruppe Cocamidopropyl Betaine, Sodium Laureth Sulfate, Sodium Cocoamphoacetate, Sodium Myreth Sulfate, Coco-Glucoside, Lauryl Glucoside, Acylglutamate, Disodium PEG-5 Laurylcitrate Sulfosuccinate und/oder Disodium Laureth Sulfosuccinate. Der Anteil der Ölphase wird gewählt im Bereich von 10 bis 20 Gew.%, der Anteil der Wasserphase im Bereich von mehr als 50 Gew.%, bezogen auf die Gesamtmasse der Zubereitung ohne Treibgase. Die Zubereitung umfasst darüber hinaus in dieser vorteilhaften Ausführungsform ca. 5 Gew.% Propyenglykol. Die 2-phasige Zubereitung wird dabei so eingestellt, dass sie eine dynamische Viskosität im Bereich von ca. 50 mPa^{*}s aufweist. Die Zubereitung wird in einem transparenten PET Aerosol vorgelegt, in dem ca. 4 bis maximal 8 Gew.% eines Treibgases, insbesondere gewählt aus Propan/Butan, enthalten sind. Der Gewichstanteil bezieht sich hinsichtlich der Treibgase hierbei auf die Gesamtmasse der Zubereitung inklusive der Treibgase.

Die erfindungsgemäße Kombination aus einem scheinbar nicht gut schäumenden, 2-phasigen Duschprodukt mit einem Ölanteil, für bessere Pflege, ermöglicht als Aerosol durch das Treibgas (vorzugsweise Propan/Butan/Isobutan Gemisch) einen besonders guten, stabilen und cremigen Schaum.

Die kosmetischen oder dermatologischen Zubereitungen gemäß der Erfindung können ferner kosmetische Hilfsstoffe und Wirkstoffe enthalten, wie sie üblicherweise in solchen Zubereitungen verwendet werden, z. B. Wirkstoffe, Konservierungsmittel, Konservierungshelfer, Bakterizide, Farbstoffe und Farbpigmente, anfeuchtende und/oder feuchthaltende Substanzen oder andere übliche Bestandteile einer kosmetischen oder dermatologischen Formulierung wie Polyole, Polymere, Schaumstabilisatoren, organische Lösungsmittel oder Silikonderivate, sofern der Zusatz die geforderten Eigenschaften hinsichtlich der Mehrphasigkeit und Schäumbarkeit nicht beeinträchtigen oder ausgeschlossen sind.

Bevorzugt umfasst die erfindungsgemäße Zubereitung ein oder mehrere Stoffe gewählt aus den Gruppen der Elektrolyte, Farbstoffe (wasserlöslich), UV-Filter, Antioxidantien, natürliche Öle (z.B. Sonneblumenöl), Vitamine (z.B. Panthenol, Niacinamide), natürliche Wirkstoffe (Q10, Süßholzextrakt, Aloe Vera, Menthol), Parfum und/oder Konservierungsmittel.

Als Elektrolyte werden erfindungsgemäß die wasserlöslichen Salze, wie insbesondere Natriumchlorid verstanden.

Aufgrund der erfindungsgemäßen Viskosität ist der Zusatz an Verdickern minimiert bzw. es wird gänzlich auf den Zusatz verzichtet. Verdicker sind Stoffe, Mittel, die die Viskosität der Zubereitung erhöhen.

Die nachfolgenden Beispiele veranschaulichen erfindungsgemäße Zubereitungen. Die Zahlenangaben sind Gewichtsanteile bezogen auf die Gesamtasse der Zubereitung, sofern nichts anderes angegeben ist.

### Beispiele Füllgut ohne Treibgas

| Handelsname, Hersteller | INCI | 1 | 2 | 3 | 4 | 5 |
|---|---|---|---|---|---|---|
| wasserlöslicher Farbstoff z.B. blau | CI 42090 | 0.09 | 0.07 | 0.00 | 0.80 | 0.09 |
| Pionier 2076, Hansen & Rosenthal | Paraffinum Liquidum | 15.00 | 19.00 | 0.00 | 14.00 | 8.00 |
| Sunflower Oil refined solvent extracted Ph. Eur. 7.0 (304031), Gustav Heess | Helianthus Annuus Seed Oil | 0.00 | 0.00 | 15.00 | 0.00 | 8.00 |
| Purolan IHD, Lanxess | Isohexadecane | 0.20 | 0.30 | 0.40 | 0.40 | 0.30 |
| Eumulgin CO 40, BASF Personal Care and Nutrition | PEG-40 Hydrogenated Castor Oil | 1.60 | 1.60 | 2.10 | 1.80 | 1.60 |
| Fragrance | Parfum | 1.00 | 1.20 | 1.10 | 1.00 | 1.00 |
| 1,2-Propylene-Glycol CARE, BASF | Propylene Glycol | 5.50 | 4.80 | 6.00 | 5.00 | 5.50 |
| Citronensäure Monohydrat, Jungbunzlauer | Citric Acid | 0.60 | 0.70 | 0.60 | 0.60 | 0.50 |
| Natrium Benzoat BP93 Pulver, PPT | Sodium Benzoate | 0.50 | 0.40 | 0.40 | 0.50 | 0.40 |
| Natriumsalicylat BP 93, Schütz & Co. | Sodium Salicylate | 0.00 | 0.25 | 0.20 | 0.30 | 0.00 |
| Wasser | Aqua | ad.q. | ad.q. | ad.q. | ad.q. | ad.q. |
| Edeta, BASF | Aqua + Trisodium EDTA (20% aktiv) | 1.90 | 1.80 | 2.10 | 2.10 | 2.00 |
| Plantacare 2000 UP, BASF Personal Care and Nutrition | Decyl Glucoside (53% aktiv) + Aqua | 0.00 | 6.00 | 0.00 | 0.00 | 2.00 |
| Tego-Betain F 50, Evonik Industries | Aqua + Cocamidopropyl Betaine (34% aktiv) + Glycerin + Sodium Chloride | 4.50 | 8.00 | 8.50 | 3.00 | 3.00 |
| Texapon N 70, BASF | Sodium Laureth Sulfate (70% aktiv) + Aqua | 18.00 | 15.00 | 14.00 | 17.00 | 12.00 |
| Uvasorb S 5, 3VSigma | Benzophenone-4 | 0.05 | 0.05 | 0.00 | 0.05 | 0.05 |

Beispielhaft wird den oben genannten Rezepturen 5% Treibgasgemisch zugesetzt, z.B. Propan/Butan/lsobutan Druckstufe 2,7, in einem transparenten Aerosolbehältnis aus PET abgefüllt und angewendet.

Die Beispielzubereitungen weisen eine dynamische Viskosität von 35 bis 80 mPa^{*}s auf (25°C).

## Patentansprüche

1. Kosmetische oder dermatologische Mehrphasenzubereitung, die durch optisch klar getrennte Phasen charakterisiert ist, umfassend
- mindestens eine Ölphase,
- mindestens eine Wasserphase, umfassend Tenside, und
- ein oder mehrere Treibgase, gewählt aus der Gruppe Propan, Butan, Isobutan und/oder deren Gemische,
**dadurch gekennzeichnet, dass**
- jede Einzelphase der Zubereitung eine dynamische Viskosität im Bereich von 20 bis 300 mPa*s (25°C) aufweist, wobei die dynamische Viskosität ermittelt wird wie in der Beschreibung angegeben und
- als Tenside eine Kombination aus anionischen und amphoteren Tensiden gewählt wird.

2. Zubereitung nach Anspruch 1 **dadurch gekennzeichnet, dass** der Anteil der Ölphase im Bereich bis zu 20 Gew.%, bezogen auf die Gesamtmasse der Zubereitung, gewählt wird.

3. Zubereitung nach Anspruch 1 oder 2 **dadurch gekennzeichnet, dass** ein oder mehrere Öle gewählt werden aus der Gruppe Paraffinum Liquidum, Isohexadecan, Isopropyl Palmitat, C12-15 Alkyl Benzoat, Sonnenblumenöl und/oder C12-13 Alkyl Lactat.

4. Zubereitung nach einem der vorstehenden Ansprüche **dadurch gekennzeichnet, dass** der Anteil an Tensiden im Bereich bis zu 20 Gew.%, bezogen auf die Gesamtmasse der Zubereitung, gewählt wird.

5. Zubereitung nach einem der vorstehenden Ansprüche **dadurch gekennzeichnet, dass** die Tenside gewählt werden aus der Gruppe Cocamidopropyl Betaine, Sodium Laureth Sulfate, Sodium Cocoamphoacetate, Sodium Myreth Sulfate, Coco-Glucoside, Lauryl Glucoside, Acylglutamate, Disodium PEG-5 Laurylcitrate Sulfosuccinate und/oder Disodium Laureth Sulfosuccinate.

6. Zubereitung nach einem der vorstehenden Ansprüche **dadurch gekennzeichnet, dass** als Tenside eine Kombination aus Cocamidopropyl Betaine und Sodium Laureth Sulfate gewählt wird.

7. Zubereitung nach einem der vorstehenden Ansprüche **dadurch gekennzeichnet, dass** die Zubereitung Propylenglykol, bevorzugt zu einem Anteil von bis 10 Gew.%, bezogen auf die Gesamtmasse der Zubereitung, enthält.

8. Zubereitung nach einem der vorstehenden Ansprüche **dadurch gekennzeichnet, dass** der Anteil an Treibgasen im Bereich von 3 bis 10 Gew.%, insbesondere im Bereich von 4 bis 8 Gew.%, bezogen auf die Gesamtmasse der Zubereitung inklusive der Treibgase, gewählt wird.

9. Zubereitung nach einem der vorstehenden Ansprüche **dadurch gekennzeichnet, dass** der Anteil an einwertigen Alkoholen, insbesondere Ethanol, Propanol und Isopropanol, in der Zubereitung weniger als 1 Gew.%, insbesondere weniger als 0,1 Gew.%, insbesondere 0 Gew.%, bezogen auf die Gesamtmasse der Zubereitung, beträgt.

10. Zubereitung nach einem der vorstehenden Ansprüche **dadurch gekennzeichnet, dass** die Zubereitung nur zwei Phasen umfasst.

11. Zubereitung nach einem der vorstehenden Ansprüche **dadurch gekennzeichnet, dass** die Viskosität jeder Einzelphase der Zubereitung im Bereich von 30 bis 150 mPa^{*}s (25°C), insbesondere im Bereich von 30 bis 100 mPa^{*}s (25°C), liegt.

12. Zubereitung nach einem der vorstehenden Ansprüche **dadurch gekennzeichnet, dass** als Tenside nur Tenside gewählt werden aus der Gruppe der anionischen und amphoteren Tenside.

13. Kit umfassend ein Behältnis und eine kosmetische oder dermatologische Mehrphasenzubereitung nach einem der vorstehenden Ansprüche
**dadurch gekennzeichnet, dass**
- die Zubereitung in einem transparentem Behältnis vorliegt,
- im Behältnis ein oder mehrere Treibgase enthalten sind, die mit der Zubereitung in Kontakt stehen, und
- die Zubereitung über eine Ausbringvorrichtung aus dem Behältnis ausgebracht werden kann.

14. Kit nach Anspruch 13 **dadurch gekennzeichnet, dass** das Behältnis Polyester, insbesondere Polyethylenterephthalat (PET), enthält bzw. daraus besteht.

15. Verwendung des Kits nach einem der vorstehenden Ansprüche 13 oder 14 als Reinigungsprodukt, insbesondere Duschprodukt oder zur Gesichtsreinigung.

## Claims

1. Cosmetic or dermatological multi-phase preparation **characterized by** optically clear separate phases, comprising
- at least one oil phase,
- at least one water phase comprising surfactants, and
- one or more propellant gases, selected from the group of propane, butane, isobutane and/or mixtures thereof, **characterized in that**
- each individual phase of the preparation has a dynamic viscosity in the range of 20 to 300 mPa^{*}s (25°C), wherein the dynamic viscosity is determined as stated in the description and
- a combination of anionic and amphoteric surfactants is selected as surfactants.

2. Preparation according to Claim 1, **characterized in that** the proportion of the oil phase is selected in the range of up to 20% by weight, based on the total mass of the preparation.

3. Preparation according to Claim 1 or 2, **characterized in that** one or more oils are selected from the group of paraffinum liquidum, isohexadecane, isopropyl palmitate, C12-15 alkyl benzoate, sunflower oil and/or C12-13 alkyl lactate.

4. Preparation according to any of the preceding claims, **characterized in that** the proportion of surfactants is selected in the range of up to 20% by weight, based on the total mass of the preparation.

5. Preparation according to any of the preceding claims, **characterized in that** the surfactants are selected from the group comprising cocamidopropyl betaine, sodium laureth sulfate, sodium cocoamphoacetate, sodium myreth sulfate, coco glucoside, lauryl glucoside, acyl glutamate, disodium PEG-5 laurylcitrate sulfosuccinate and/or disodium laureth sulfosuccinate.

6. Preparation according to any of the preceding claims, **characterized in that** a combination of cocamidopropyl betaine and sodium laureth sulfate is selected as surfactants.

7. Preparation according to any of the preceding claims, **characterized in that** the preparation comprises propylene glycol, preferably at a proportion of up to 10% by weight, based on the total mass of the preparation.

8. Preparation according to any of the preceding claims, **characterized in that** the proportion of propellant gases is selected in the range of 3 to 10% by weight, particularly in the range of 4 to 8% by weight, based on the total mass of the preparation including the propellant gases.

9. Preparation according to any of the preceding claims, **characterized in that** the proportion of monohydric alcohols, particularly ethanol, propanol and isopropanol, in the preparation is less than 1% by weight, particularly less than 0.1% by weight, especially 0% by weight, based on the total mass of the preparation.

10. Preparation according to any of the preceding claims, **characterized in that** the preparation comprises only two phases.

11. Preparation according to any of the preceding claims, **characterized in that** the viscosity of each individual phase of the preparation is in the range of 30 to 150 mPa*s (25°C), especially in the range of 30 to 100 mPa*s (25°C).

12. Preparation according to any of the preceding claims, **characterized in that** the surfactants selected are surfactants only from the group of anionic and amphoteric surfactants.

13. Kit comprising a container and a cosmetic or dermatological multi-phase preparation according to any of the preceding claims, **characterized in that**
- the preparation is in a transparent container,
- one or more propellant gases, which are in contact with the preparation, are present in the container, and
- the preparation can be dispensed from the container via a dispensing device.

14. Kit according to Claim 13, **characterized in that** the container comprises or consists of polyester, especially polyethylene terephthalate (PET).

15. Use of the kit according to either of preceding Claims 13 or 14 as a cleansing product, particularly a shower product or for facial cleansing.

## Revendications

1. Préparation multiphasique cosmétique ou dermatologique, qui est **caractérisée par** des phases séparées optiquement transparentes, comprenant
- au moins une phase huileuse,
- au moins une phase aqueuse comprenant des tensioactifs, et
- un ou plusieurs gaz propulseurs choisis dans le groupe propane, butane, isobutane et/ou les mélanges de ceux-ci,
**caractérisée en ce que**
- chaque phase individuelle de la préparation présente une viscosité dynamique dans la plage de 20 à 300 mPa^{*}s (25°C), la viscosité dynamique étant déterminée comme indiqué dans la description, et
- on choisit en tant que tensioactifs une combinaison de tensioactifs anioniques et amphotères.

2. Préparation selon la revendication 1, **caractérisée en ce que** la proportion de la phase huileuse est choisie dans plage allant jusqu'à 20 % en poids par rapport à la masse totale de la préparation.

3. Préparation selon la revendication 1 ou 2, **caractérisée en ce qu'**on choisit une ou plusieurs huiles dans le groupe Paraffinum Liquidum, isohexadécane, palmitate d'isopropyle, benzoate d'alkyle en C12-15, huile de tournesol et/ou lactate d'alkyle en C12-13.

4. Préparation selon l'une des revendications précédentes, **caractérisée en ce que** la proportion des tensioactifs est choisie dans la plage allant jusqu'à 20 % en poids par rapport à la masse totale de la préparation.

5. Préparation selon l'une des revendications précédentes, **caractérisée en ce que** les tensioactifs sont choisis dans le groupe cocoamidopropyl-bétaïne, laureth-sulfate de sodium, cocoamphoacétate de sodium, myreth-sulfate de sodium, coco-glucoside, lauryl-glucoside, glutamate d'acyle, laurylcitrate et sulfosuccinate disodique PEG-5 et/ou laureth-sulfosuccinate disodique.

6. Préparation selon l'une des revendications précédentes, **caractérisée en ce qu'**on choisit en tant que tensioactifs une combinaison de cocoamidopropyl-bétaïne et de laureth-sulfate de sodium.

7. Préparation selon l'une des revendications précédentes, **caractérisée en ce que** la préparation contient du propylèneglycol, de préférence selon une proportion allant jusqu'à 10 % en poids par rapport à la masse totale de la préparation.

8. Préparation selon l'une des revendications précédentes, **caractérisée en ce que** la proportion des gaz propulseurs est choisie dans la plage de 3 à 10 % en poids, en particulier dans la plage de 4 à 8 % en poids par rapport à la masse totale de la préparation, y compris les gaz propulseurs.

9. Préparation selon l'une des revendications précédentes, **caractérisée en ce que** la proportion des monoalcools, en particulier l'éthanol, le propanol et l'isopropanol, dans la préparation est inférieure à 1 % en poids, en particulier inférieure à 0,1 % en poids, en particulier de 0 % en poids par rapport à la masse totale de la préparation.

10. Préparation selon l'une des revendications précédentes, **caractérisée en ce que** la préparation ne comprend que deux phases.

11. Préparation selon l'une des revendications précédentes, **caractérisée en ce que** la viscosité de chaque phase individuelle de la préparation est dans la plage de 30 à 150 mPa^{*}s (25°C), en particulier dans la plage de 30 à 100 mPa^{*}s (25°C).

12. Préparation selon l'une des revendications précédentes, **caractérisée en ce qu'**on choisit en tant que tensioactifs uniquement des tensioactifs du groupe des tensioactifs anioniques et amphotères.

13. Kit comprenant un récipient et une préparation multiphasique cosmétique ou dermatologique selon l'une des revendications précédentes, **caractérisé en ce que**
- la préparation est présente dans un récipient transparent,
- le récipient contient un ou plusieurs gaz propulseurs, qui sont en contact avec la préparation, et
- la préparation peut être éjectée du récipient par l'intermédiaire d'un dispositif d'éjection.

14. Kit selon la revendication 13, **caractérisé en ce que** le récipient contient du polyester, en particulier du poly(téréphtalate d'éthylène) (PET) ou en est constitué.

15. Utilisation du kit selon l'une des revendications précédentes 13 ou 14 comme produit nettoyant, en particulier comme produit pour la douche ou pour le nettoyage du visage.
